# EUROPEAN PATENT APPLICATION

(11) **EP 3 012 325 A1**
(43) Date of publication of application: **27.04.2016**
(21) Application number: 15199896.0
(22) Date of filing: 10.11.2011
(51) Int. Cl.: C12P 7/18, C12N 1/32, C07K 14/33

(54) **MICROORGANISMS FOR 1,3-PROPANEDIOL PRODUCTION USING HIGH GLYCERINE CONCENTRATION**

(30) Priority: 10.11.2010 US 412162 P; 10.11.2010 EP 10306234
(62) Divisional of application: 11779682.1
(71) Applicant: Metabolic Explorer, 63360 Saint Beauzire (FR)
(72) Inventor: FIGGE, Rainer, 63450 LE CREST (FR)
(74) Representative: Regimbeau

(57) **Abstract**

The present invention is related to a population of *Clostridium acetobutylicum* useful for the production of 1,3-propanediol (PDO), wherein said population comprises at least one strain of a *Clostridium acetobutylicum* sp. comprising mutations selected among the mutations identified in table 1, wherein relative percentages of said mutations are selected among specific genes.

## Description

The present invention concerns a new modified microorganism for the production of 1,3-propanediol. This microorganism is adapted for growth and production of 1,3-propanediol from a culture medium with high glycerine content and specifically with a high concentration of industrial glycerine. The invention also concerns culture conditions of said adapted microorganisms and process for the production of 1,3-propanediol. The invention concerns, finally, 1,3-propanediol produced by the modified microorganism and its applications.

### BACKGROUND OF THE INVENTION

1,3-propanediol (PDO), also called trimethylene glycol or propylene glycol, is one of the oldest know fermentation products. It was originally identified as early as 1881 by August Freund in a glycerine fermented culture containing *Clostridium pasteurianum.* PDO is a typical product of glycerine fermentation and has been found in anaerobic conversions of other organic substrates. Only very few organisms, all of them bacteria, are able to form it. They include enterobacteria of the genera *Klebsiella* (*K. pneumoniae*), *Enterobacter* (*E. agglomerans*) and *Citrobacter* (*C. freunddi*), *Lactobacilli* (*L. brevis* and *L. buchneri*) and *Clostridia* of the *C*. *butyricum* and the *C*. *pasteurianum* group.

PDO, as a bifunctional organic compound, may potentially be used for many synthesis reactions, in particular as a monomer for polycondensations to produce polyesters, polyethers, polyurethanes, and in particular, polytrimethylene terephtalate (PTT). These structure and reactivity features lead to several applications in cosmetics, textiles (clothing fibers or flooring) or plastics (car industry and packing or coating).

PDO can be produced by different chemical routes but they generate waste stream containing extremely polluting substances and the cost of production is high. Thus, chemically produced PDO can not compete with the petrochemically available diols like 1,2-ethanediol, 1,2-propanediol and 1,4-butanediol. To increase this competitiveness, in 1995, DuPont started a research program for the biological conversion of glucose to PDO. Although this process is environmentally friendly it has the disadvantage to i) use vitamin B12 a very expensive cofactor and ii) be a discontinuous process due to the instability of the producing strain.

Due to the availability of large amounts of glycerine produced by the bio-diesel industry, a continuous, vitamin-B12-free process with a higher carbon yield would on the contrary, be advantageous.

It is known in the art that PDO can be produced from glycerine, an unwanted by-product of the biodiesel production that contains roughly 80-85% of glycerine mixed with salts and water.

*C*. *butyricum* was previously described as being able to grow and produce PDO from industrial glycerine in batch and two-stage continuous fermentation (Papanikolaou *et al.,* 2000). However, at the highest glycerine concentration, the maximal PDO titer obtained was 48.1 g.L⁻¹ at a dilution rate of 0.02 h⁻¹, meaning a productivity of 0.9 g.L⁻¹.h⁻¹. The cultures were conducted with a maximum glycerine concentration in the fed medium of 90g.L⁻¹ and in the presence of yeast extract, a costly compound containing organic nitrogen that is known by the man skilled in the art to help increase bacterial biomass production.

Application WO2006/128381 discloses the use of this glycerine for the production of PDO with batch and fed-batch cultures using natural PDO producing organisms such as *Klebsiella pneumoniae, C. butyricum* or *C*. *pasteuricum.* Furthermore, the medium used in WO2006/128381 also contains yeast extract. As described in this patent application, the maximal productivity reached was comprised between 0.8 and 1.1g.l⁻¹.h⁻¹.

The performance of a *C*. *acetobutylicum* strain modified to contain the vitamin B12-independent glycerine-dehydratase and the PDO-dehydrogenase from *C. butyricum,* called *"C. acetobutylicum* DG1 pSPD5" has been described in Gonzalez-Pajuelo *et al.,* 2005. This strain originally grows and produces PDO in a fed medium containing up to 120 g.l⁻¹ of pure glycerine. In addition, analyses in a fed medium containing a maximum of 60 g.l⁻¹ of pure or industrial glycerine did not point out to any differences. These results have been obtained in presence of yeast extract. Moreover,no test was performed with concentrations of industrial glycerine higher than 60g.l⁻¹. When comparing a wild-type *C*. *butyricum* to the modified microorganism "*C*. *acetobutylicum* DG1 pSPD5", a globally similar behaviour was observed.

In patent application PCT/EP2010/056078 the inventors have described a process to adapt the strain of *C*. *acetobutylicum* DG1 pSPD5 such as described in Gonzalez-Pajuelo *et al.* (2005) to grow in a medium with a high concentration of industrial glycerine and without yeast extract. The resulting strain is able to produce PDO in medium containing up to 120 g.l⁻¹ of industrial glycerine with a titer up to 53.5 g.L⁻¹ of PDO, a yield up to 0.53 g.g⁻¹ and a productivity up to 2.86 g.L⁻¹.h⁻¹.

In the present patent application, the inventors highlight the main genetics modifications of the adapted microorganism useful for the production of PDO, such as obtained after adaptation in presence of high concentration of industrial glycerine.

### BRIEF DESCRIPTION OF THE INVENTION

The present invention concerns a population of *Clostridium acetobutylicum* useful for the production of 1,3-propanediol (PDO), wherein said population comprises at least one strain of a *Clostridium acetobutylicum* sp. comprising mutations selected among the mutations identified in Table 1.

Said differently, the present invention concerns a population of *Clostridium acetobutylicum* useful for the production of 1,3-propanediol (PDO), wherein said population comprises at least one strain of a *Clostridium acetobutylicum* sp. comprising mutations selected among the mutations identified in Table 1, wherein relative percentages of said mutations are selected among the following gene families:

| Gene family and function | Minimum % |
|---|---|
| Transcription translation regulation | 12-15 |
| Transporters | 10-12 |
| Hypothetical proteins | 8-11 |
| Energy metabolism | 7-10 |
| Intergenic | 7-10 |
| Carbohydrate metabolism | 5-7 |
| Membrane proteins | 2-5 |
| Nucleic acid metabolism | 2-5 |
| Amino acid metabolism | 1-3 |
| Cell division | 1-3 |
| Sporulation | 1-3 |
| Cell adhesion | 0-1 |
| Cellulase | 0-1 |
| Glycerol metabolism | 0-1 |
| Lipid metabolism | 0-1 |
| Proteases/Peptidases | 0-1 |
| Cell motility | 0-1 |

Particularly, the population of the invention comprises at least one strain of *Clostridium acetobutylicum* selected among the group consisting of:
- strain DG1 pSPD5 PD0001VE05c01 deposited at CNCM under accession number I-4378;
- strain DG1 pSPD5 PD0001VE05c05 deposited at CNCM under accession number I-4379;
- strain DG1 pSPD5 PD0001VE05c07 deposited at CNCM under accession number I-4380.
CNCM means "Collection Nationale de Cultures de Microorganismes" at the Pasteur Institute, Paris.

In a particular embodiment of the invention, the population comprises the above strains further mutated with at least one of the following point mutations:
- C is replaced with T at locus CA_C0175, position 198989 in the *Clostridium acetobutylicum* genome, coding for a predicted sugar phosphate isomerase, homolog of an eukaryotic glucokinase regulator (carbohydrate metabolism)
- G is replaced with A at locus CA_C1300, position 1444099 in the *Clostridium acetobutylicum* genome, coding for an RNA polymerase sigma factor RPOD ( transcription and translation regulation)
- C is replaced with T at locus CA_C2670, position 2787387 in the *Clostridium acetobutylicum* genome, coding for a Glu-tRNAGln amidotransferase subunit A (transcription and translation regulation)
- C is replaced with T at locus CA_C3339, position 3512658 in the *Clostridium acetobutylicum* genome, coding for an ATPase component of an ABC transporter (two ATPase domains)
- C is replaced with T at locus CA_C1610, position 1752341 in the *Clostridium acetobutylicum* genome, coding for a branched-chain amino acid permease (transporter).

The present invention also concerns a method for the production of 1,3-propanediol, comprising culturing a population of *Clostridium acetobutylicum* useful for the production of 1,3-propanediol (PDO) according to the invention in a culture medium comprising glycerine as sole source of carbon and recovering the 1,3-propanediol produced from the culture medium.

### DETAILLED DESCRIPTION OF THE INVENTION

### Population of Clostridium acetobutylicum useful for the production of 1,3-propanediol (PDO)

A population of *Clostridium acetobutylicum* useful for the production of 1,3-propanediol means one or more strains of *Clostridium acetobutylicum* genetically modified for the production of 1,3-propanediol from glycerine as sole source of carbon. Such strains are known in the art and disclosed, particularly, in applications WO200104324 and WO2008052595. The population according to the invention may be a combination of several strains, the majority of which comprising the mutations according to the invention, as well as a single strain, and particularly strain DG1 pSPD5 PD0001VE05c01, DG1 pSPD5 PD0001VE05c05 or DG1 pSPD5 PD0001VE05c07 deposited at CNCM under accession numbers I-4378, I-4379, I-4380 respectively, or strain DG1 pSPD5 PD0001VE05c08.

Mutations are changes of nucleotides in the strain genome, more particularly SNPs ("Single Nucleotide Polymorphisms"), identified when compared to the mother strain DG1 pSPD5 PD0001VT. Said strain is disclosed in WO200104324 and is derived from strain ATCC824 which genome sequence has been published (Nölling *et al.,* 2001).

Mutations can occur in coding or non-coding sequences. These mutations can be synonymous wherein there is not modification of the corresponding amino acid or non-synonymous wherein the corresponding amino acid is altered. Synonymous mutations do not have any impact on the function of translated proteins, but may have an impact on the regulation of the corresponding genes or even of other genes, if the mutated sequence is located in a binding site for a regulator factor. Non-synonymous mutations may have an impact on the function of the translated protein as well as on regulation depending the nature of the mutated sequence.

The population of *Clostridium acetobutylicum* useful for the production of 1,3-propanediol may preferably comprise one of those deposited strains comprising additional modifications, at least one of the following modifications:
- C replaced with T at locus CA_C0175, position 198989 in the *Clostridium acetobutylicum* genome, coding for a predicted sugar phosphate isomerase, homolog of an eukaryotic glucokinase regulator (carbohydrate metabolism)
- G replaced with A at locus CA_C1300, position 1444099 in the *Clostridium acetobutylicum* genome, coding for an RNA polymerase sigma factor RPOD ( transcription and translation regulation)
- C replaced with T at locus CA_C2670, position 2787387 in the *Clostridium acetobutylicum* genome, coding for a Glu-tRNAGln amidotransferase subunit A (transcription and translation regulation)
- C replaced with T at locus CA_C3339, position 3512658 in the *Clostridium acetobutylicum* genome, coding for an ATPase component of an ABC transporter (two ATPase domains) - C replaced with T at locus CA_C1610, position 1752341 in the *Clostridium acetobutylicum* genome, coding for a branched-chain amino acid permease (transporter).

It may preferably comprise any combinations of these mutations, comprising 1, 2, 3, 4 or 5 of these mutations.

The population of strains of the invention is capable of growing on a medium comprising up to 120 g.L⁻¹ of glycerine and more particularly of industrial glycerine.

The strains of the population of the invention may be obtained using standard techniques of mutagenesis and/or gene replacement in *Clostridium,* such as disclosed in application WO2008040387 which contents are incorporated herein by reference.

The person skilled in the art may start from one of the strains disclosed in applications WO200104324 and WO2008052595 as well as use one of the strains c01, c05 or c07 deposited at CNCM under accession numbers I-4378, I-4379, I-4380 respectively, and introduce additional mutations.

In a preferred embodiment, the population of the invention comprises strain DG1 pSPD5 PD0001VE05c08, which mutations are identified in Table 1. The person skilled in the art knows how to introduce the mutations into a *Clostridium* strain to generate a strain similar to strain DG1 pSPD5 PD0001VE05c08, starting from one of strains DG1 pSPD5 PD0001VE05c01, DG1 pSPD5 PD0001VE05c05 or DG1 pSPD5 PD0001VE05c07, deposited at CNCM under accession numbers I-4378, I-4379, I-4380 respectively and using standard gene replacement and recombination techniques.

### Culture medium comprising glycerine

An "appropriate culture medium" or a "culture medium" refers to a culture medium optimized for the growth and the diol-production of the *Clostridium* strains or population. The fermentation process is generally conducted in reactors with a synthetic, particularly inorganic, culture medium of known defined composition adapted to the *Clostridium* species used and containing glycerine.

The term "synthetic medium" means a culture medium comprising a chemically defined composition on which organisms are grown. In the culture medium of the present invention, glycerine is advantageously the single source of carbon.

The terms "glycerine" and 'glycerol" are synonymous and used interchangeably in this invention to refer to the same molecule.

In a particular embodiment, glycerine is added to the medium in the form of glycerine composition comprising at least 50% of glycerine, preferably at least 85% of glycerine.

Advantageously, the glycerine used in the culture medium of the invention is industrial glycerine. "Industrial glycerine" means a glycerine product obtained from an industrial process without substantial purification. Industrial glycerine can also be designated as "raw glycerine". Industrial glycerine comprises more than 70% of glycerine, preferably more than 80%, water and impurities such as mineral salts and fatty acids. The maximum content of glycerine in industrial glycerine is generally 90%, more generally about 85%.

Industrial processes form which industrial glycerine is obtained are, inter alia, manufacturing methods where fats and oils, particularly fats and oils of plant origin, are processed into industrial products such as detergent or lubricants. In such manufacturing methods, industrial glycerine is considered as a by-product.

In a preferred embodiment, the industrial glycerine used in the culture medium of the invention is a by-product of biodiesel production.

In a particular embodiment, the industrial glycerine is a by-product from biodiesel production and comprises known impurities of glycerine obtained from biodiesel production, comprising about 80 to 85% of glycerine with salts, water and some other organic compounds such as fatty acids. Industrial glycerine obtained from biodiesel production has not been subjected to further purification steps.

Preferably, the culture medium comprises high concentrations of glycerine.

The terms "high glycerine content" or "high concentration of glycerine" means more than 90 g.L⁻¹ of glycerine in the culture medium. In preferred embodiments, the concentration is comprised between 90 and 200 g.L⁻¹ of glycerine, more particularly between 90 and 140 g/L of glycerine, preferably about 120 g.L⁻¹ of glycerine.

According to a preferred embodiment, in the method for the production of 1,3-propanediol according to the invention, the glycerine concentration in the culture medium is comprised between 90 and 120 g/L glycerine, and is preferably about 105g/L of glycerine.

Preferably, the culture medium is a synthetic medium without addition of organic nitrogen.

Such culture media are disclosed in the art, particularly in PCT/EP2010/056078 filed on 05/05/2010 and PCT/EP2010/064825 filed on 5/10/2010, which contents are incorporated herein by reference.

### Culturing the microorganisms

In the method of the invention, the production is advantageously done in a batch, fed-batch or continuous process. Culturing microorganisms at industrial scale for the production of 1,3-propanediol is known in the art, particularly disclosed in PCT/EP2010/056078 filed on 05/05/2010 and PCT/EP2010/064825 filed on 5/10/2010, which content are incorporated herein by reference.

### 1,3-propanediol recovery

The method for the production of 1,3-propanediol according to the invention comprises culturing a population of one of claims 1 to 4 in a culture medium comprising glycerine as sole source of carbon, and recovering the 1,3-propanediol produced from the culture medium. In a preferred embodiment, the 1,3-propanediol is further purified.

Methods for recovering and eventually purifying 1,3-propanediol from a fermentation medium are known to the skilled person. 1,3-propanediol may be isolated by distillation. In most embodiments, 1,3-propanediol is distilled from the fermentation medium with a by-product, such as acetate, and then further purified by known methods.

A particular purification method is disclosed in applications WO2009/068110 and WO 2010/037843, which content is incorporated herein by reference.

### FIGURES

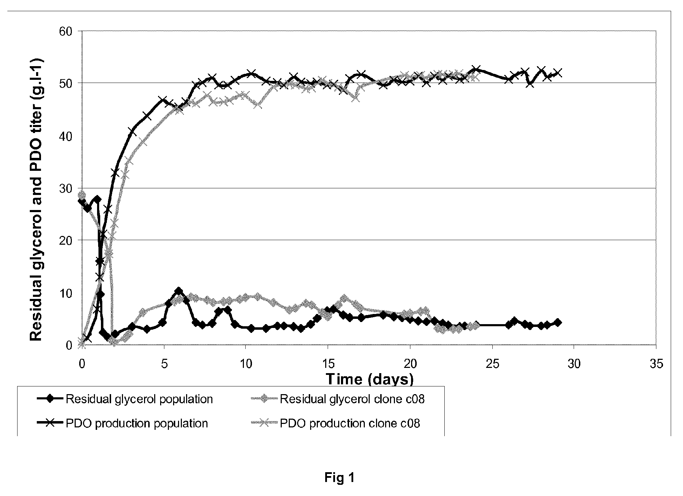

**Figure 1** describes the evolution of 1,3-propanediol production and glycerine consumption of the population and clone c08 during the chemostat cultures from inoculation up to D = 0.06h⁻¹.

### EXAMPLES

### EXAMPLE 1 Isolation of clones from the evolved population

Clone isolation was performed on agar plates starting from a growing flask culture of the population strain *Clostridium acetobutylicum* DG1 pSPD5 PD0001VE05. The synthetic media used for flask culture contained per liter of deionized water : glycerine, 30g; KH₂PO₄, 0.5g; K₂HPO₄, 0.5g; MgSO₄, 7H₂O, 0.2g; CoCl₂ 6H₂O, 0.01g; acetic acid, 99.8%, 2.2ml; NH₄Cl, 1.65g; MOPS, 23.03g, biotin, 0.16mg; *p*-amino benzoic acid, 32mg; FeSO₄, 7H₂O, 0.028g; resazurin, 1mg and cysteine, 0.5g. The pH of the medium was adjusted to 6.5 with NH₄OH 6N.

Different media were used for isolation on agar plates : synthetic agar medium (the same as described above) with either commercial glycerine or raw glycerine and CGM (Clostridial Growth Medium) agar medium which contains per liter of deionized water : commercial or raw glycerine, 30g; yeast extract, 5g; KH₂PO₄, 0.75; K₂HPO₄, 0.75g; MgSO₄, 7H₂O, 0.4g; asparagine, 2g; (NH₄)₂SO₄, 2g; NaCl, 1g; MnSO₄, H2O, 10mg; FeSO₄, 7H₂O, 10mg; MOPS, 23.03g; resasurin, 1mg and cysteine, 15g. The pH of the medium was adjusted to 6.6 with NH₄OH 3N.

Cells were plated from a flask culture (Table 2) in four different ways:
- on agar plates of synthetic medium with commercial glycerine ;
- on agar plates of synthetic medium with raw glycerine ;
- on agar plates of rich medium with commercial glycerine ;
- on agar plates of rich medium with raw glycerine.

Isolated clones were considered pure after three subsequent subcultures on agar plates. Pure clones were then transferred into liquid rich medium which contained either commercial or raw glycerine (Table 2). Subsequently, growing liquid cultures were conserved on glycerine 20% at -80°C until further characterization.

Clones were then characterized in the following way:
- Measurement of viability after conservation : evaluation of growth rate of cells on synthetic medium ;
- Evaluation of growth and metabolism : measurement of OD₆₂₀ₙₘ during culture and PDO/glycerine yield on synthetic medium ;
- Genetic evaluation : PCR analysis to confirm the genotype of the strain;
- Chemostat culture to compare the performances of isolated clones with those of the population (example 2) ;
- gDNA extraction for sequence analysis of the clones (example 3).

**Table 2: Synthetic agar media and liquid media used for the isolation of 4 clones from the population.**

| **Clone number** | **Agar media for isolation** | **Liquid media for clone culture before conservation** |
|---|---|---|
| c01 | Synthetic medium with commercial glycerine | Rich medium with commercial glycerine |
| c05 | Rich medium with raw glycerine | Rich medium with commercial glycerine |
| c07 | Synthetic medium with commercial glycerine | Rich medium with raw glycerine |
| c08 | Rich medium with commercial glycerine | Rich medium with raw glycerine |

### EXAMPLE 2 Performances of clone c08 in a chemostat culture with high concentrations of raw glycerine

### Bacterial strain:

Isolated clone of *C*. *acetobutylicum* strain DG1 pSPD5 PD0001VE05 (strain was 1/ cured from pSOL1 2/ transformed with plasmid pSPD5 harbouring *dhaB1*, *dhaB2* and *dhaT* genes, ie 1,3-propanediol operon, and 3/ evolved on high concentrations of raw glycerine). The isolation protocol was described in example 1.

### Culture media:

The synthetic media used for clostridia batch cultivations contained per liter of deionized water: glycerine, 30g; KH₂PO₄, 0.5g ; K₂HPO₄, 0.5g ; MgSO₄, 7H₂O, 0.2g ; CoCl₂ 6H₂O, 0.01g ; H₂SO₄, 0.1ml ; NH₄Cl, 1.5g ; biotin, 0.16mg ; *p*-amino benzoic acid, 32mg and FeSO₄, 7H₂O, 0.028g. The pH of the medium was adjusted to 6.3 with NH₄OH 3N. Commercial glycerine purchased from Sigma (purity 99.5%) was used for batch cultivation. The feed medium for continuous cultures contained per liter of tap water: raw glycerine, 105g ; KH₂PO₄, 0.5g ; K₂HPO₄, 0.5g ; MgSO₄, 7H₂O, 0.2g ; CoCl₂ 6H₂O, 0.026g ; NH₄Cl, 1.5g ; biotin, 0.16mg ; *p*-amino benzoic acid, 32mg ; FeSO₄, 7H₂O, 0.04g ; antifoam, 0,05ml ; ZnSO₄, 7H₂O, 8mg ; CuCl₂, 2H₂O, 4mg ; MnSO₄, H₂O, 40mg ; H₃BO₃, 2mg ; Na₂MoO₄, 2H₂O, 0.8mg. Medium pH was not adjusted in this case. Raw glycerine, from the transesterification process for biodiesel, was supplied by Novance (Venette, France) and had the following purity : glycerine 84.8% (w/w).

### Experimental set-up:

Continuous cultures were performed in a 5l bioreactor Tryton (Pierre Guerin, France) with a working volume of 2000ml. The culture volume was kept constant at 2000ml by automatic regulation of the culture level. Cultures were stirred at 200 RPM, the temperature was set to 35°C and pH maintained constant at 6.5 by automatic addition of NH₄OH 5.5N. The POR measurement (mV) was controlled during the entire culture. To create anaerobic conditions, the sterilized medium in the vessel was flushed with sterile O₂-free nitrogen for one hour at 60°C and flushed again until 35°C was attained (flushing during 2 hours). The bioreactor gas outlet was protected from oxygen by a pyrogallol arrangement (Vasconcelos *et al,* 1994). After sterilisation the feed medium was also flushed with sterile O₂-free nitrogen until room temperature was attained and maintained under nitrogen at 200 mbar to avoid O₂ entry.

### Batch and continuous cultures process:

The process used to evaluate has been described in patent application PCT/EP2010/056078 (example 2).

A culture growing in a 100ml flask on synthetic medium (the same as described above for batch culture but with addition of acetic acid, 2.2 g.L⁻¹ and MOPS, 23.03g.L⁻¹) taken at the end of exponential growth phase was used as inoculum (5% v/v).

Cultures were first grown batchwise. At the early exponential growth phase we performed a pulse of commercial glycerine: For the pulse synthetic medium (the same as described for batch culture) with 105 g.L⁻¹ raw glycerine was added at a static flow rate during 3 hours (i.e. an addition of 18 g.L⁻¹ of glycerine). Then the growth continued batchwise and before the end of the exponential growth phase the continuous feeding started with a dilution rate of 0.025 h⁻¹: The feed medium contains 105 g.L⁻¹ of raw glycerine. 8-10 days after inoculation of the bioreactor and after 3 residence times the dilution rate was increased from 0.025 h⁻¹ to 0.060h⁻¹ by different stages: Increase of 0.01 h⁻¹ units in 48 hours - no change for 24-hours - increase of 0.01 h⁻¹ units in 48 hours - no change for 24hours - increase of 0.015 h⁻¹ unit in 48 hours. After that, stabilisation of the culture was followed by 1,3-propanediol production and glycerine consumption (Figure 1) using the HPLC protocol described below. Particularly we waited until the concentration of residual glycerine was as low as possible.

The overall performances of c08 clone are presented in Table 3 and compared with performances of the population under the same conditions and with performances of the strain *C*. *acetobutylicum* DG1 pSPD5 PD0001VT such as described in Gonzalez-Pajuelo *et al.* (2005).

### Analytical procedures:

Cell concentration was measured turbidimetrically at 620nm and correlated with cell dry weight determined directly. Glycerine, 1,3-propanediol, ethanol, butanol, acetic and butyric acids concentrations were determined by HPLC analysis. Separation was performed on a Biorad Aminex HPX-87H column and detection was achieved by refractive index.

Operating conditions were as follows: mobile phase sulphuric acid 0.5mM; flow rate 0.5ml/min, temperature, 25°C.

**Table 3: performances of the C. acetobutylicum DG1 pSPD5 population PD0001VE05 (mean data from 4 chemostats), of clone c08 PD0001VE05c08. The feed medium contained 105g.L⁻¹ of raw glycerine, dilution rate was 0.060h⁻¹ and 0.025h⁻¹. Values correspond to the average of samples analyzed after at least 3 residences times at dilution rate of 0.060h⁻¹.**

| | Average and standard deviation for clone c08 PD0001VE05c08 | Average and standard deviation of the population PD0001VE05 | Average and standard deviation of the strain PD0001VT (Gonzalez-Pajuelo et al. 2005) |
|---|---|---|---|
| Feed glycerol (g.l⁻¹) | 105.49 +/- 1.07 | 104.21 +/- 1.36 | 58.54 |
| 1,3-propanediol (g.l⁻¹) | 51.30 +/- 0.54 | 50.45 +/- 1.00 | 29.76 |
| Y_{1,3-PDO} (g.g⁻¹) | 0.50 +/- 0.01 | 0.53 +/- 0.01 | 0.50 |
| Q_{1,3PDO} (q.l⁻¹.h⁻¹) | 3.05 +/- 0.03 | 3.18 +/- 0.21 | 1.49 |
| | | | |
| Dilution rate (h⁻¹) | 0.059 +/- 0.001 | 0.063 +/- 0.004 | 0.05 |
| Residual glycerol (g.l⁻¹) | 3.72 +/- 1.62 | 4.82 +/- 1.82 | 0 |
| Biomass (g.l⁻¹) | 1.52 +/- 0.55 | 2.09 +/- 0.15 | 1.64 |
| Acetic acid (g.l⁻¹) | 2.67 +/- 0.26 | 2.09 +/- 0.27 | NI |
| Butyric acid (g.l⁻¹) | 10.53 +/- 0.38 | 10.98 +/- 0.37 | NI |

| | | | |
|---|---|---|---|
| Y_{1,3-PDO} : PDO yield (g/g of glycerol consumed) Q_{1,3PDO} : PDO volumetric productivity NI: no information. The PD0001VT strain can not grow in a medium lacking yeast extract. | | | |

These results show that the adapted population of *C*. *acetobutylicum* DG1 pSPD5 is able to grow on higher concentrations of industrial glycerine and thus exhibits a better titer and productivity of PDO on industrial glycerine, than the non adapted strain *C*. *acetobutylicum* DG1 pSPD5 PD0001VT from Gonzalez-Pajuelo *et al.* (2005) which can not grow in a medium lacking yeast extract.

### Example 3

### Genomic DNA extraction

Genomic DNA from strains PD0001VT, PD0001VE05, PD0001VE05c01, PD0001VE05c05, PD0001VE05c07 and PD0001VE05c08 was extracted using Qiagen Genomic kit 500G (Qiagen, Inc., Valencia, CA). Briefly, cells were grown anaerobically respectively in rich or synthetic glycerine medium (as described in example 1 and 2) in penicillin vials (70 mL) to late exponential phase (A₆₂₀ 1.5 to 2.0). Strictly anaerobic conditions were maintained throughout cell lysis. Cells were collected and washed twice in SET buffer (25% sucrose, 0.05 M Tris-HCl, 0.05 M EDTA). Cell pellets were suspended in 11 mL B1 kit buffer with 44 µL RNase, 30 mg/mL lysozyme and 100 µg/mL proteinase K. The mixtures were incubated at 37°C for 45 min, centrifuged and supernatants were used for DNA extraction according to the Qiagen DNA purification kit instructions. The DNAs were then suspended in 50 µL of 10 mM Tris-HCl (pH8.0).

### Sequencing analysis

Genomes of the native DG1 pSPD5 PD0001VT strain and the evolved population DG1 pSPD5 PD0001VE05 were sequenced using the Roche GS FLX technology. The sequencing project was performed by Eurofins Genomics MWG/ Operon (ZA de Courtabeauf-9 Avenue de la Laponie, 91978 Les Ulis Cedex) with for each strain 1 Long-Tag paired end libraries (8 Kb), generation of sequence and scaffolding of the contigs with GS FLX Titanium series chemistry using a half run (max. 600 000 reads, max 180 000-300 000 true paired end reads).

Isolated clones from the evolved population were sequenced using the comparative genomic sequencing (CGS) method developed by NimbleGen (Roche NimbleGen Inc. 500 S. Rosa Rd. Madison WI 53719). The CGS analysis was performed in two phases: in phase 1, regions of genomic difference were identified by a comparative hybridization of DNA of the native strain and the evolved clones. In phase 2, only the identified regions of genomic differences were sequenced so as to produce a set of fully characterized single nucleotide polymorphisms (SNPs).

### SNP analysis

Bioinformatics and SNP analysis of the evolved population were performed by Eurofins Genomics MWG / Operon. For this analysis, the read sets of both strains were separately mapped to the Genbank reference sequence (*Clostridium acetobutylicum* ATCC 824 http://www.ncbi.nlm.nih.gov/nuccore/AE001437) using the software gsMapper (Roche 454, V2.3). Three SNPs files were delivered comparing DG1 pSPD5 PD0001VT to ATCC824, DG1 pSPD5 PD0001VE05 to ATCC824 and DG1 pSPD5 PD0001VT to DG1 pSPD5 PD0001VE05. Unique SNPs between the native and the evolved strains are presented below. Low coverage (<25) and low variant frequency (<85%) were removed resulting in 160 unique SNPs distributed in 17 families according to the KEGG database used for the family group annotations.

SNP analysis of the isolated clones was performed by NimbleGen (Roche). The SNP files were delivered comparing native DG1 pSPD5 PD0001VT to DG1 pSPD5 PD0001VE05c01, DG1 pSPD5 PD0001VE05c05, DG1 pSPD5 PD0001VE05c07 or DG1 pSPD5 PD0001VE05c08 using Genbank reference sequence (*Clostridium acetobutylicum* ATCC 824 http://www.ncbi.nlm.nih.gov/nuccore/AE001437).

The sequence results are presented in Table 1 which contains the following information:
- RefStart: the start position within the reference sequence, where the difference occurs
- RefNuc: the reference nucleotide sequence at the difference location
- VarNuc: the differing nucleotide sequence at the difference location
- VarFreq: the percentage of different reads versus total reads that fully span the difference location
- Type: Lists whether or not an SNP is found within an annotated gene, or between annotated genes. SNPs in genes are designated as coding. SNPs between genes are designated as intergenic
- AA change: categorizes coding SNPs base on whether or not they change the amino acid sequence of a protein. S indicates synonymous SNPs (no amino acid change). N indicates nonsynonymous SNPs (altered amino acid). FC (Frame-Change) indicates a modification in protein translation because of insertion or deletion of a nucleotide
- ORIG_AA: the amino acid associated with the reference sequence for the corresponding SNP position
- SNP_AA: the amino acid associated with the test sequence, for the corresponding SNP position
- Locus Tag: locus tag of the corresponding gene from Genbank
- Function: the function of the gene as described in Genbank
- Family: the family of the gene from KEGG

**Table 1: Mutations between native and evolved strains. Mutations were first identified in the adapted population and then presence of each mutation was verified in isolated clones (four last columns: Y for presence and N for absence of mutation).**

| **Ref Start** | **Ref Nuc** | **Var Nuc** | **Var Freq** | **Type** | **AA cha.** | **YRIG AA** | **SNP AA** | **Locus Tag** | **Function** | **Family** | **c01** | **c05** | **c07** | **c08** |
|---|---|---|---|---|---|---|---|---|---|---|---|---|---|---|
| 15594 | C | T | >99% | C | N | L | P | CA_C0009 | Uncharacterized conserved protein, ortholog of YRXA B.subtilis | Hypothetical proteins | Y | Y | Y | Y |
| 21339 | G | A | >99% | I | | | | | I | I | Y | Y | Y | Y |
| 25354 | G | A | >99% | C | N | G | S | CA_C0017 | Seryl-tRNA synthetase | Transcription translation regulation | Y | Y | Y | Y |
| 29516 | G | A | >99% | C | N | G | D | CA_C0020 | MDR-type permease | Transporters | Y | Y | Y | Y |
| 31667 | C | T | >99% | C | N | A | V | CA_C0021 | Seryl-tRNA synthetase (serine-tRNA ligase) | Transcription translation regulation | Y | Y | Y | Y |
| 35547 | G | A | >99% | C | S | R | R | CA_C0024 | Membrane protein, related to Actinobacillus protein (1944168) | Membrane proteins | Y | Y | Y | Y |
| 43029 | C | T | >99% | C | N | A | V | CA_C0032 | Transcriptional regulator TetR/AcrR family | Transcription translation regulation | Y | Y | Y | Y |
| 52503 | A | G | >99% | C | S | A | A | CA_C0039 | DNA segregation ATPase FtsK/SpoIIIE family protein, contains FHA domain | Cell division | Y | Y | Y | Y |
| 76769 | C | T | >99% | C | S | F | F | CA_C0066 | ABC transporter, ATP-binding protein | Transporters | Y | Y | Y | Y |
| 143022 | G | A | >99% | C | S | S | S | CA_C0135 | Hypothetical protein, CF-23 family | Hypothetical proteins | Y | Y | Y | Y |
| 198989 | C | T | >99% | C | N | T | I | CA_C0175 | Predicted sugar phosphate isomerase, homolog of eucaryotic glucokinase regulator | Carbohydrate metabolism | Y | Y | Y | Y |
| 219199 | C | T | >99% | C | N | M | I | CA_C0193 | Uncharacterized conserved membrane protein, affecting LPS biosynthesis | Membrane proteins | Y | Y | Y | Y |
| 220222 | T | C | >99% | C | N | N | S | CA_C0194 | Glycosyltransferase involved in cell wall biogenesis | Carbohydrate metabolism | Y | Y | Y | Y |
| 265152 | G | A | >99% | C | N | G | S | CA_C0234 | PTS system, fructoso-specific IIBC component | Carbohydrate metabolism | Y | Y | Y | Y |
| 286308 | G | A | >99% | C | N | A | T | CA_C0256 | Nitrogenase molybdenum-iron protein, alpha chain (nitrogenase component I) gene nifD | Energy metabolism | Y | Y | Y | Y |
| 347024 | G | A | >99% | C | N | A | T | CA_C0291 | FUSION: methionine synthase I (cobalamin dependent) and 5,10 methylenetetrahydrofolate reductase | Amino acid metabolism | Y | Y | Y | Y |
| 364263 | A | G | >99% | I | | | | | I | I | Y | Y | Y | Y |
| 454074 | G | A | >99% | C | N | E | K | CA_C0390 | Cystathionine gamma-synthase | Amino acid metabolism | Y | Y | Y | Y |
| 541268 | C | T | >99% | C | N | T | I | CA_C0471 | GrpE protein HSP-70 cofactor | Transcription translation regulation | Y | Y | Y | Y |
| 601197 | A | G | >99% | I | | | | | I | I | Y | Y | Y | Y |
| 621074 | C | T | >99% | C | N | P | L | CA_C0534 | Phosphoenolpyruvate synthase (gene pps) | Carbohydrate metabolism | Y | Y | Y | Y |
| 656050 | T | C | >99% | C | N | S | P | CA_C0566 | Malate dehydrogenase | Energy metabolism | Y | Y | Y | Y |
| 668495 | A | G | >99% | C | S | I | I | CA_C0578 | Cobalamine-dependent methionine synthase I (methyltransferase and cobalamine-binding domain) | Amino acid metabolism | Y | Y | Y | Y |
| 723431 | - | A | >99% | I | | | | | I | I | N | N | N | N |
| 794841 | C | T | >99% | C | N | T | I | CA_C0688 | 1-acyl-sn-glycerine-3-phosphate acyltransferase | Glycerine metabolism | Y | Y | Y | Y |
| 817479 | C | T | >99% | C | N | A | V | CA_C0706 | Endo-1,4-beta glucanase (fused to two ricin-B-like domains) | Cellulase | Y | Y | Y | Y |
| 819542 | C | T | >99% | C | N | P | L | CA_C0707 | RNA polymerase sigma-54 factor | Transcription translation regulation | Y | Y | Y | Y |
| 951584 | A | G | >99% | C | N | I | V | CA_C0823 | Predicted membrane protein | Membrane proteins | Y | Y | Y | Y |
| 977563 | G | A | >99% | I | | | | | I | I | Y | Y | Y | Y |
| 978671 | C | T | >99% | C | S | C | C | CA_C0850 | Nitroreductase family protein | Energy metabolism | Y | Y | Y | Y |
| 991021 | T | C | >99% | C | N | V | A | CA_C0861 | ABC-type multidrug transport system, ATPase component | Transporters | Y | Y | Y | Y |
| 991449 | G | A | >99% | C | N | G | R | CA_C0861 | ABC-type multidrug transport system, ATPase component | Transporters | Y | Y | Y | Y |
| 1019710 | G | A | >99% | C | N | V | | CA_C0888 | Phosphoglycerine transferase MdoB related protein, alkaline phosphatase superfamily | Glycerine metabolism | Y | Y | Y | Y |
| 1068817 | T | C | >99% | C | N | L | S | CA_C0925 | TPR-repeat-containing protein | Hypothetical proteins | Y | Y | Y | Y |
| 1113238 | G | A | >99% | C | N | N | S | CA_C0967 | Probably membrane protein | Membrane proteins | Y | Y | Y | Y |
| 1223725 | G | A | >99% | C | N | A | T | CA_C1072 | Fe-S oxidoreductase | Energy metabolism | Y | Y | Y | Y |
| 1254865 | T | A | >99% | C | N | Y | N | CA_C1086 | Transcriptional regulators of NagC/XyIR family | Transcription translation regulation | Y | Y | Y | Y |
| 1299105 | A | G | >99% | C | N | M | T | CA_C1133 | Phage related protein, YonE B.subtilis homolog | Hypothetical proteins | Y | Y | Y | Y |
| 1309504 | C | T | >99% | C | N | M | I | CA_C1143 | Exodeoxyribonuclease V, Alpha subunit, RecD | Cell division | Y | Y | Y | Y |
| 1324897 | A | G | >99% | C | S | P | P | CA_C1166 | Hypothetical protein | Hypothetical proteins | Y | Y | Y | Y |
| 1366058 | T | A | >99% | C | N | N | K | CA_C1223 | DNA Polymerase III Alpha chain (dnaE) | Transcription translation regulation | Y | Y | Y | Y |
| 1424502 | A | G | >99% | C | N | Y | C | CA_C1280 | Transcriptional regulator of heat shock genes, HrcA | Transcription translation regulation | Y | Y | Y | Y |
| 1444099 | G | A | >99% | C | N | G | R | CA_C1300 | RNA polymerase sigma factor RPOD | Transcription translation regulation | Y | Y | Y | Y |
| 1540446 | A | G | >99% | C | S | L | L | CA_C1396 | Phosphoribosylamine-glycine ligase | Nucleic acid metabolism | Y | Y | Y | Y |
| 1554592 | A | G | >99% | C | N | K | R | CA_C1408 | Phospho-beta-glucosidase | Carbohydrate metabolism | Y | Y | Y | Y |
| 1644651 | A | - | >99% | I | | | | | I | I | Y | Y | Y | Y |
| 1770126 | A | G | >99% | C | N | I | V | CA_C1628 | DNA gyrase A subunit | Transcription translation regulation | Y | Y | Y | Y |
| 1778678 | G | A | >99% | C | N | A | T | CA_C1636 | Uncharacterized protein, homolog of B. firmus (2654481) | Hypothetical proteins | Y | Y | Y | Y |
| 1805186 | G | A | >99% | C | N | V | I | CA_C1661 | Predicted secreted nucleic acid binding protein | Nucleic acid metabolism | Y | Y | Y | Y |
| 1821699 | G | A | >99% | C | N | A | T | CA_C1673 | Large subunit of NADH-dependent glutamate synthase | Energy metabolism | Y | Y | Y | Y |
| 1916660 | A | G | >99% | C | N | N | S | CA_C1771 | Uncharacterized protein, ykrl B.subtilis homolog | Hypothetical proteins | Y | Y | Y | Y |
| 1948050 | C | T | >99% | I | | | | | I | I | Y | Y | Y | Y |
| 2037205 | G | A | >99% | C | S | C | C | CA_C1886 | Uncharacterized phage related protein | Hypothetical proteins | Y | Y | Y | Y |
| 2114483 | A | G | >99% | C | N | V | A | CA_C2003 | Predicted permease | Transporters | Y | Y | Y | Y |
| 2123888 | T | C | >99% | C | S | L | L | CA_C2010 | Predicted Fe-S oxidoreductase | Energy metabolism | Y | Y | Y | Y |
| 2171503 | C | T | >99% | C | N | D | N | CA_C2068 | Sporulation factor spoIIM, uncharacterized membrane protein | Sporulation | Y | Y | Y | Y |
| 2231570 | C | - | >99% | C | FC | | | CA_C2137 | Cation transport P-type ATPase | Transporters | N | N | N | Y |
| 2294764 | G | A | >99% | C | N | T | I | CA_C2201 | Hypothetical protein | Hypothetical proteins | Y | Y | Y | Y |
| 2299326 | C | G | >99% | C | N | S | T | CA_C2205 | Flagellar hook-associated protein FliD | Cell motility | Y | Y | Y | Y |
| 2307214 | C | T | >99% | C | N | G | R | CA_C2215 | Flagellar switch protein FliY, contains CheC-like domain | Cell motility | Y | N | Y | Y |
| 2342826 | G | C | >99% | C | N | P | A | CA_C2247 | Site-specific recombinase, DNA invertase Pin homolog | Transcription translation regulation | Y | Y | Y | Y |
| 2392178 | C | T | >99% | C | N | V | . | CA_C2288 | Acyl-protein synthetase, luxE | Lipid metabolism | Y | Y | Y | Y |
| 2450006 | C | T | >99% | C | S | P | P | CA_C2340 | DNA mismatch repair protein mutS, YSHD B.subtilis ortholog | Transcription translation regulation | Y | Y | Y | Y |
| 2477825 | C | T | >99% | C | S | S | S | CA_C2367 | Uncharacterized protein containing predicted cell adhesion domain and ChW-repeats | Cell adhesion | Y | Y | Y | Y |
| 2493211 | T | C | >99% | C | S | H | H | CA_C2385 | Hypothetical protein | Hypothetical proteins | Y | Y | Y | Y |
| 2595349 | G | A | >99% | C | N | A | V | CA_C2486 | Transcriptional regulator, MarR/EmrR family | Transcription translation regulation | Y | Y | Y | Y |
| 2693354 | C | T | >99% | C | N | E | K | CA_C2588 | Glycosyltransferase | Carbohydrate metabolism | Y | Y | Y | Y |
| 2787387 | C | T | >99% | C | N | M | I | CA_C2670 | Glu-tRNAGln amidotransferase subunit A | Transcription translation regulation | Y | Y | Y | Y |
| 2833384 | T | C | >99% | C | N | I | V | CA_C2709 | Electron transfer flavoprotein alpha-subunit | Energy metabolism | Y | Y | Y | Y |
| 2836979 | G | A | >99% | C | N | A | V | CA_C2713 | AT-rich DNA-binding protein | Transcription translation regulation | Y | Y | Y | Y |
| 2901642 | C | T | >99% | C | N | V | . | CA_C2770 | Amino acid transporter | Transporters | Y | Y | Y | Y |
| 2969858 | G | A | >99% | C | N | M | I | CA_C2838 | Predicted nucleotide-binding protein, YjeE family | Transcription translation regulation | Y | Y | Y | Y |
| 3001642 | G | A | >99% | C | S | L | L | CA_C2867 | FoF1-type ATP synthase alpha subunit | Energy metabolism | Y | Y | Y | Y |
| 3032956 | T | C | >99% | C | N | H | R | CA_C2898 | Stage II sporulation protein R | Sporulation | Y | Y | Y | Y |
| 3140918 | T | C | >99% | I | | | | | I | I | Y | Y | Y | Y |
| 3174743 | G | A | >99% | C | S | D | D | CA_C3032 | Galactose mutarotase related enzyme | Carbohydrate metabolism | Y | N | Y | Y |
| 3251276 | G | C | >99% | C | N | T | S | CA_C3099 | Pseudouridylate synthase, TRUA | Nucleic acid metabolism | Y | Y | Y | Y |
| 3337937 | G | - | >99% | I | | | | | I | I | N | N | N | N |
| 3392124 | G | A | >99% | C | N | G | R | CA_C3242 | Uncharacterized Fe-S protein, PfIX (pyruvate formate lyase activating protein) homolog | Energy metabolism | Y | Y | Y | Y |
| 3462380 | C | T | >99% | C | S | N | N | CA_C3297 | D-alanyl-D-alanine carboxypeptidase family hydrolase, YODJ B.subtilis ortholog | Hypothetical proteins | Y | Y | Y | Y |
| 3509372 | C | T | >99% | C | S | E | E | CA_C3335 | Short-chain alcohol dehydrogenase family enzyme | Energy metabolism | Y | Y | Y | Y |
| 3512658 | C | T | >99% | C | S | Y | Y | CA_C3339 | ATPase component of ABC transporter (two ATPase domains) | Transporters | Y | Y | Y | Y |
| 3518240 | T | C | >99% | C | S | Y | Y | CA_C3345 | Transcriptional regulator, AcrR family | Transcription translation regulation | Y | Y | Y | Y |
| 3541557 | T | C | >99% | C | N | I | V | CA_C3363 | Hypothetical protein | Hypothetical proteins | Y | Y | Y | Y |
| 3565291 | C | T | >99% | C | N | T | I | CA_C3387 | Pectate lyase | Cellulase | Y | Y | Y | Y |
| 3576865 | T | C | >99% | C | N | H | R | CA_C3392 | NADH-dependent butanol dehydrogenase | Energy metabolism | Y | Y | Y | Y |
| 3583724 | C | T | >99% | I | | | | | I | I | Y | Y | Y | Y |
| 3608511 | C | T | >99% | C | S | S | S | CA_C3422 | Sugar:proton symporter (possible xylulose) | Transporters | Y | Y | Y | Y |
| 3614985 | C | T | >99% | C | S | K | K | CA_C3428 | 6Fe-6S prismane cluster-containing protein | Energy metabolism | Y | Y | Y | Y |
| 3674358 | T | C | >99% | I | | | | | I | I | Y | Y | Y | Y |
| 3707038 | T | C | >99% | C | S | L | L | CA_C3510 | Membrane associated methyl-accepting chemotaxis protein (with HAMP domain) | Membrane proteins | Y | Y | Y | Y |
| 3747653 | G | A | >99% | C | N | A | V | CA_C3551 | Na+ ABC transporter (ATP-binding protein), NATA | Transporters | Y | Y | Y | Y |
| 3821135 | C | T | >99% | C | S | N | N | CA_C3617 | Uncharacterized membrane protein, YHAG B.subtilis homolog | Hypothetical proteins | Y | Y | Y | Y |
| 3850220 | A | G | >99% | C | N | I | T | CA_C3650 | HD-GYP domain containing protein | Proteases/Peptidases | Y | Y | Y | Y |
| 3921509 | C | T | >99% | C | N | V | I | CA_C3716 | Lon-like ATP-dependent protease | Proteases/Peptidases | Y | Y | Y | Y |
| 239312 | G | A | 98% | C | N | E | K | CA_C0214 | Endoglucanase, aminopeptidase M42 family | Cellulase | Y | Y | Y | Y |
| 244251 | C | T | 98% | I | | | | | I | I | Y | Y | Y | Y |
| 2410308 | G | A | 98% | C | S | L | L | CA_C2306 | Sporulation-specific sigma factor F | Sporulation | Y | Y | Y | Y |
| 3656844 | G | A | 98% | C | N | A | T | CA_C3459 | Homolog of cell division GTPase FtsZ, diverged | Cell division | Y | Y | Y | Y |
| 3823060 | A | G | 98% | C | N | V | A | CA_C3620 | Amino acid (probably glutamine) ABC transporter, periplasmic binding protein component | Transporters | Y | Y | Y | Y |
| 3838496 | C | T | 98% | C | N | G | R | CA_C3637 | Oligopeptide ABC transporter, permease component | Transporters | N | N | N | N |
| 914 | G | A | 97% | C | N | G | R | CA_C0001 | DNA replication initiator protein, ATPase | Cell division | Y | Y | Y | Y |
| 27240 | C | T | 97% | C | N | R | Q | CA_C0019 | Transcriptional regulator, AcrR family | Transcription translation regulation | N | Y | N | Y |
| 36341 | G | A | 97% | C | N | D | N | CA_C0025 | Deoxycytidine triphosphate deaminase | Nucleic acid metabolism | N | Y | N | N |
| 299266 | G | A | 97% | C | N | D | N | CA_C0267 | L-lactate dehydrogenase | Energy metabolism | Y | N | Y | Y |
| 376886 | G | A | 97% | C | S | K | K | CA_C0322 | Sensory protein, containing EAL-domain | Transcription translation regulation | Y | Y | Y | Y |
| 474915 | G | A | 97% | C | N | G | D | CA_C0408 | DNA segregation ATP-ase FtsK/SpoIIIE (three ATPases), contains FHA domain | Cell division | Y | Y | Y | Y |
| 599925 | G | A | 97% | C | N | R | K | CA_C0519 | Dihydroorotase | Nucleic acid metabolism | Y | Y | Y | Y |
| 723433 | G | A | 97% | I | | | | | I | I | N | N | N | N |
| 723434 | GT | - | 97% | I | | | | | I | I | N | N | N | N |
| 723871 | A | G | 97% | C | N | I | M | CA_C0622 | Polyphosphate kinase | Energy metabolism | Y | Y | Y | Y |
| 809008 | C | T | 97% | C | S | L | L | CA_C0699 | Spore photoproduct lyase, splB | Sporulation | Y | Y | Y | Y |
| 846466 | G | T | 97% | C | N | A | S | CA_C0731 | FUSION: Nucleoside-diphosphate-sugar epimerase and GAF domain | Nucleic acid metabolism | Y | Y | Y | Y |
| 1717948 | G | A | 97% | C | N | V | I | CA_C1572 | Fructose-1,6-bisphosphatase (YYDE B.subtils ortholog) | Carbohydrate metabolism | Y | Y | Y | Y |
| 2004797 | C | T | 97% | C | N | S | N | CA_C1852 | Magnesium and cobalt transport protein | Transporters | Y | Y | Y | Y |
| 2134058 | G | A | 97% | C | S | A | A | CA_C2020 | Molybdopterin bioSthesis enzyme, MoeA, fused to molibdopterin-binding domain | Energy metabolism | Y | Y | Y | Y |
| 2331746 | G | A | 97% | C | N | G | R | CA_C2237 | ADP-glucose pyrophosphorylase | Lipid metabolism | Y | Y | Y | Y |
| 2391588 | G | A | 97% | C | N | P | L | CA_C2288 | Acyl-protein Sthetase, luxE | Lipid metabolism | Y | Y | Y | Y |
| 2452705 | C | T | 97% | C | N | C | Y | CA_C2341 | Collagenase family protease | Proteases/Peptidases | Y | Y | Y | Y |
| 2739459 | T | C | 97% | C | N | I | V | CA_C2630 | Uncharaterized conserved protein, YOME B.subtilis ortholog | Hypothetical proteins | Y | Y | Y | Y |
| 2775979 | C | T | 97% | C | N | A | T | CA_C2660 | Pyruvate carboxylase, PYKA | Carbohydrate metabolism | Y | Y | Y | Y |
| 2813985 | G | - | 97% | I | | | | | I | I | N | N | N | N |
| 3082247 | C | T | 97% | C | N | L | F | CA_C2948 | ATPase components of ABC transporter with duplicated ATPase domains (second domain is inactivated) | Transporters | Y | Y | Y | Y |
| 3242900 | G | C | 97% | C | N | V | L | CA_C3088 | NtrC family transcriptional regulator, ATPase domain fused to two PAS domains | Transcription translation regulation | Y | N | N | Y |
| 3442855 | T | C | 97% | C | N | M | V | CA_C3282 | ABC-type multidrug/protein/lipid transport system, ATPase component | Transporters | Y | Y | Y | Y |
| 3498584 | C | T | 97% | C | N | L | F | CA_C3327 | Amino acid ABC-type transporter, ATPase component | Transporters | Y | Y | Y | Y |
| 3643224 | G | A | 97% | C | S | L | L | CA_C3447 | Protein-disulfide isomerases DsbC/DsbG | Sporulation | Y | Y | Y | Y |
| 3663477 | - | T | 97% | C | FC | | | CA_C3464 | Uncharacterized conserved protein (fragment) | Hypothetical proteins | N | N | N | N |
| 204202 | G | A | 96% | C | N | G | E | CA_C0180 | Oligopeptide ABC transporter, ATP-binding protein | Transporters | Y | Y | Y | Y |
| 803682 | C | T | 96% | C | N | T | I | CA_C0695 | Altronate oxidoreductase | Carbohydrate metabolism | Y | Y | Y | Y |
| 892875 | G | A | 96% | C | N | M | I | CA_C0770 | Glycerine uptake facilitator protein, permease | Glycerine metabolism | Y | Y | Y | Y |
| 1009389 | C | T | 96% | C | N | P | S | CA_C0879 | ABC-type polar amino acid transport system, ATPase component | Transporters | Y | Y | Y | Y |
| 1690355 | C | T | 96% | C | S | G | G | CA_C1546 | Pyrimidine-nucleoside phosphorylase | Nucleic acid metabolism | Y | Y | Y | Y |
| 1752341 | C | T | 96% | C | N | G | R | CA_C1610 | Branched-chain amino acid permease | Transporters | Y | Y | Y | Y |
| 3217481 | A | C | 96% | C | S | L | L | CA_C3067 | Predicted membrane protein | Membrane proteins | Y | Y | Y | Y |
| 3238489 | T | C | 96% | C | S | S | S | CA_C3086 | Protein containing cell adhesion domain | Cell adhesion | Y | Y | Y | Y |
| 447460 | A | - | 95% | I | | | | | I | I | N | N | N | N |
| 670931 | G | A | 95% | C | S | N | N | CA_C0578 | Cobalamine-dependent methionine synthase I (methyltransferase and cobalamine-binding domain) | Amino acid metabolism | N | Y | Y | Y |
| 994575 | G | A | 95% | C | N | A | T | CA_C0864 | Histidine kinase-like ATPase | Transcription translation regulation | Y | Y | Y | Y |
| 3657101 | A | - | 95% | C | FC | | | CA_C3459 | Homolog of cell division GTPase FtsZ, diverged | Cell division | N | N | N | N |
| 1142263 | T | - | 94% | C | FC | | | CA_C0995 | Predicted membrane protein | Membrane proteins | N | N | N | N |
| 1823156 | G | A | 94% | C | S | E | E | CA_C1674 | Small subunit of NADPH-dependent glutamate synthase | Amino acid metabolism | Y | Y | Y | Y |
| 1989117 | C | T | 94% | C | N | R | K | CA_C1837 | Mismatch repair protein MutS, ATPase | Transcription translation regulation | Y | Y | Y | Y |
| 3481651 | G | A | 94% | C | S | S | S | CA_C3311 | TPR-repeat domain fused to glycosyltransferase | Carbohydrate metabolism | Y | Y | Y | Y |
| 126942 | G | A | 93% | C | N | E | K | CA_C0116 | Carbone-monoxide dehydrogenase, beta chain | Energy metabolism | Y | Y | Y | Y |
| 302716 | - | T | 93% | C | FC | | | CA_C0270 | Hypothetical protein | Hypothetical proteins | N | N | N | N |
| 2551103 | G | A | 93% | C | S | S | S | CA_C2434 | Membrane associate histidine kinase with HAMP domain | Transcription translation regulation | Y | N | Y | Y |
| 1834077 | C | T | 92% | C | N | S | L | CA_C1684 | TYPA/BIPA type GTPase | Energy metabolism | Y | Y | Y | Y |
| 3927304 | G | A | 92% | I | | | | | I | I | Y | N | Y | N |
| 786649 | - | T | 91% | C | FC | | | CA_C0680 | Predicted membrane protein | Membrane proteins | N | N | N | N |
| 2640439 | C | T | 91% | C | N | E | K | CA_C2532 | Protein containing ChW-repeats | Cell adhesion | Y | Y | Y | Y |
| 3601904 | A | - | 91% | C | FC | | | CA_C3415 | ABC-type multidrug/protein/lipid transport system, ATPase component | Transporters | N | N | N | N |
| 838350 | A | - | 89% | C | FC | | | CA_C0723 | Transcriptional regulator, AcrR family | Transcription translation regulation | N | N | N | N |
| 3721023 | G | A | 89% | C | S | S | S | CA_C3523 | Hypothetical protein, CF-7 family | Hypothetical proteins | Y | Y | Y | Y |
| 803924 | G | A | 88% | C | N | A | T | CA_C0695 | Altronate oxidoreductase | Carbohydrate metabolism | Y | Y | Y | Y |
| 3478420 | C | T | 87% | C | N | G | E | CA_C3309 | Predicted membrane protein | Membrane proteins | N | Y | N | Y |
| 3853836 | T | C | 87% | C | N | N | D | CA_C3652 | Acetolactate synthase | Amino acid metabolism | Y | Y | Y | Y |
| 244464 | C | T | 86% | C | N | S | L | CA_C0220 | Hypothetical protein | Hypothetical proteins | Y | Y | Y | Y |
| 899104 | G | A | 86% | C | N | M | I | CA_C0776 | NCAIR mutase (PurE)-related protein | Nucleic acid metabolism | Y | N | Y | N |
| 658665 | T | - | 85% | C | FC | | | CA_C0569 | SACPA operon antiterminator (sacT) | Transcription translation regulation | N | N | N | N |

### REFERENCES

1. González-Pajuelo M, Meynial-Salles I, Mendes F, Andrade JC, Vasconcelos I, and Soucaille P. 2005. Metabolic engineering of Clostridium acetobutylicum for the industrial production of 1,3-propanediol from glycerol. Metabolic Engineering 7: 329-336.
2. González-Pajuelo M, Meynial-Salles I, Mendes F, Soucaille P. and Vasconcelos I. 2006. Microbial conversion of a natural producer, Clostridium butyricum VPI 3266, and an engineered strain, Clostridium acetobutylicum DG (pSPD5). Applied and Environmental Microbiology, 72: 96-101.
3. Nölling J, Breton G, Omelchenko MV, Makarova KS, Zeng Q, Gibson R, Lee HM, Dubois J, Qiu D, Hitti J, Wolf YI, Tatusov RL, Sabathe F, Doucette-Stamm L, Soucaille P, Daly MJ, Bennett GN, Koonin EV, Smith DR. 2001. Genome sequence and comparative analysis of the solvent-producing bacterium Clostridium acetobutylicum. Journal of bacteriology 183(16):4823 - 4838
4. Papanikolaou S, Ruiz-Sanchez P, Pariset B, Blanchard F and Fick M. 2000. High production of 1,3-propanediol from industrial glycerol by a newly isolated Clostridium butyricum strain. Journal of Biotechnology. 77: 191-208.
5. Vasconcelos I, Girbal L, Soucaille P. 1994. Regulation of carbon and electron flow in Clostridium acetobutylicum grown in chemostat culture at neutral pH on mixtures of glucose and glycerol. Journal of bacteriology. 176(5): 1443-1450.

## Claims

1. A population of *Clostridium acetobutylicum* useful for the production of 1,3-propanediol (PDO), wherein said population comprises at least one strain of a *Clostridium acetobutylicum* sp. comprising mutations selected among the mutations identified in Table 1.

2. The population of claim 1, wherein it comprises at least one strain of *Clostridium acetobutylicum* selected among the group consisting of
- strain DG1 pSPD5 PD0001VE05c01 deposited at CNCM under accession number I-4378
- strain DG1 pSPD5 PD0001VE05c05 deposited at CNCM under accession number I-4379
- strain DG1 pSPD5 PD0001VE05c07 deposited at CNCM under accession number I-4380.

3. The population of claim 1 or 2, wherein the strains are further mutated with at least one of the following mutations
- C replaced with T at locus CA_C0175, position 198989 in the *Clostridium acetobutylicum* genome, coding for a predicted sugar phosphate isomerase, homolog of an eukaryotic glucokinase regulator (carbohydrate metabolism)
- G replaced with A at locus CA_C1300, position 1444099 in the *Clostridium acetobutylicum* genome, coding for an RNA polymerase sigma factor RPOD ( transcription and translation regulation)
- C replaced with T at locus CA_C2670, position 2787387 in the *Clostridium acetobutylicum* genome, coding for a Glu-tRNAGln amidotransferase subunit A (transcription and translation regulation)
- C replaced with T at locus CA_C3339, position 3512658 in the *Clostridium acetobutylicum* genome, coding for an ATPase component of an ABC transporter (two ATPase domains)
- C replaced with T at locus CA_C1610, position 1752341 in the *Clostridium acetobutylicum* genome, coding for a branched-chain amino acid permease (transporter).

4. A method for the production of 1,3-propanediol, comprising culturing a population of one of claims 1 to 3 on a culture medium comprising glycerine as a sole source of carbon and recovering the 1,3-propanediol produced from the culture medium.

5. The method of claim 4, wherein the 1,3-propanediol is further purified.

6. The method of one of claims 4 or 5, wherein the glycerine concentration in the culture medium is comprised between 90 and 120 g/L glycerine, preferably about 105g/L of glycerine.

7. The method of one of claims from 4 to 6, wherein the glycerine is provided by industrial glycerine.

8. The method of claim 7, wherein the industrial glycerine is a by-product of biodiesel production.

9. The method of one of claims 4 to 9, wherein the culture medium is a synthetic medium, without addition of organic nitrogen.
